# EUROPEAN PATENT SPECIFICATION

(11) **EP 1 232 254 B1**
(45) Date of publication and mention of the grant of the patent: **24.09.2008**
(21) Application number: 00991956.4
(22) Date of filing: 13.11.2000
(51) Int. Cl.: C12N 9/68, A61K 38/48

(54) **PROCESS FOR THE PRODUCTION OF A REVERSIBLY INACTIVE ACIDIFIED PLASMIN COMPOSITION**
VERFAHREN ZUR HERSTELLUNG EINER REVERSIBEL INAKTIVEN, ANGESäUERTEN PLASMINZUSAMMENSETZUNG
PROCEDE DE PRODUCTION D'UNE COMPOSITION DE PLASMINE ACIDIFIEE INACTIVEE DE MANIERE REVERSIBLE

(30) Priority: 13.11.1999 US 438331
(43) Date of publication of application: 21.08.2002
(73) Proprietor: Talecris Biotherapeutics, Inc., Research Triangle Park NC 27709 (US)
(72) Inventor: DADD, Christopher, Cary, NC 27511 (US); STENLAND, Christopher, J., Cary, NC 27513 (US); KENT, Jonathan, D., Holly Springs, NC 27540 (US); KORNEYEVA, Marina, N., Raleigh, NC 27606 (US); BAUMBACH, George, A., Knightdale, NC 27545 (US); COOK, Scott, A., Garner, NC 27529 (US); BRADLEY, Rita, T., Cary, NC 27513 (US); NOVOKHATNY, Valery, Raleigh, NC 27612 (US); VILLINES, Tanette, B., Raleigh, NC 27604 (US)
(74) Representative: HOFFMANN EITLE
(86) International application number: PCT/US2000/042143
(87) International publication number: WO 2001/036611

(56) References cited:
- EP-A- 0 399 321
- EP-A- 0 835 931
- WO-A-93/15189
- GB-A- 904 478
- GB-A- 2 090 599
- US-A- 3 950 513
- US-A- 4 115 551
- US-A- 4 259 448
- US-A- 5 096 637
- US-A- 5 237 050
- US-A- 5 288 489
- US-A- 5 328 996
- US-A- 5 879 923
- CASTELLINO F J ET AL: "Rabbit plasminogen and plasmin isozymes." METHODS IN ENZYMOLOGY. UNITED STATES 1976, vol. 45, 1976, pages 273-286, XP008030352 ISSN: 0076-6879
- BINDER B R ET AL: "PURIFICATION AND CHARACTERIZATION OF HUMAN VASCULAR PLASMINOGEN ACTIVATOR DERIVED FROM BLOOD VESSEL PERFUSATES" JOURNAL OF BIOLOGICAL CHEMISTRY, vol. 254, no. 6, 1979, pages 1998-2003, XP002279534 ISSN: 0021-9258
- ROBBINS K C ET AL: "HUMAN PLASMINOGEN AND PLASMIN" PERLMANN, GERTRUDE E. AND LAZLO LORAND (EDITED BY). METHODS IN ENZYMOLOGY, vol. XIX, 1970, pages 184-199, XP008030353 PRESS: NEW YORK, N.Y., U.S.A.;LONDON ENGLAND 1970
- PATENT ABSTRACTS OF JAPAN vol. 1997, no. 07, 31 July 1997 (1997-07-31) -& JP 09 065895 A (NITTO BOSEKI CO LTD), 11 March 1997 (1997-03-11)
- KLINE D L: "THE PURIFICATION AND CRYSTALLIZATION OF PLASMINOGEN (PROFIBRINOLYSIN)" JOURNAL OF BIOLOGICAL CHEMISISTRY, vol. 204, 1953, pages 949-955, XP002307256
- DATABASE WPI Section Ch, Week 199511 Derwent Publications Ltd., London, GB; Class B04, AN 1995-082184 XP002307257 & WO 95/04077 A1 (GREEN CROSS CORP) 9 February 1995 (1995-02-09)
- PATENT ABSTRACTS OF JAPAN vol. 014, no. 270 (C-0727), 12 June 1990 (1990-06-12) & JP 02 078633 A (GREEN CROSS CORP:THE), 19 March 1990 (1990-03-19)

## Description

### Field of the Invention

The present invention relates generally to a method of producing plasmin and more particularly to a method of purifying and isolating the plasmin under conditions which stabilize against degradation.

### Background

Fibrin is a white insoluble fibrous protein formed from fibrinogen by the action of thrombin. In the clotting of blood, fibrin forms the structural scaffold of a thrombus, which is a clot of blood formed within a blood vessel that remains attached to its place of origin. Under normal conditions the blood clotting system is maintained in equilibrium and the fibrin deposits, are dissolved by the fibrinolytic enzyme system. Unfortunately, events such as vascular damage, activation/stimulation of platelets, and activation of the coagulation cascade may disturb the equilibrium, which can result in thrombosis or the blockage of a blood vessel by a blood clot.

Intravascular thrombosis is one of the most frequent pathological events accounting for greater than 50% of all deaths as well as a variety of other serious clinical problems. Most spontaneously developing vascular obstructions are due to the formation of intravascular blood clots, also known as thrombi. Small fragments of a clot may detach from the body of the clot and travel through the circulatory system to lodge in distant organs and initiate further clot formation. Myocardial infarction, occlusive stroke, deep venous thrombosis (DVT) and peripheral arterial disease are well known consequences of thromboembolic phenomena.

Plasminogen activators are currently the favored agents employed in thrombolytic therapy, all of which convert plasminogen to plasmin and promote fibrinolysis by disrupting the fibrin matrix (M.A. Creager and V.J. Dzau, Vascular Diseases of the Extremities, ppgs. 1398 - 1406 in Harrison's Principles of Internal Medicine, 14th ed., Fauci et al, editors, McGraw-Hill Co., New York, 1998; the contents of which is incorporated herein by reference in its entirety).

The most widely used plasminogen activators include a recombinant form of tissue-type plasminogen activator (tPA), urokinase (UK) and streptokinase (SK), as well as a new generation of plasminogen activators selected for improved pharmacokinetics and fibrin-binding properties. All of these plasminogen activators, however, by virtue of their mechanism of action, act indirectly and require an adequate supply of their common substrate, plasminogen, at the site of the thrombus to effect lysis.

UK and tPA convert plasminogen to plasmin directly by cleaving the Arg⁵⁶⁰-Val⁵⁶¹ peptide bond. The resulting two polypeptide chains of plasmin are held together by two interchain disulfide bridges. The light chain of 25kDa carries the catalytic center and is homologous to trypsin and other serine proteases. The heavy chain (60kDa) consists of five triple-loop kringle structures with highly similar amino acid sequences. Some of these kringles contain so-called lysine-binding sites that are responsible for plasminogen and plasmin interaction with fibrin, alpha 2-antiplasmin or other proteins. SK and staphylokinase activate plasminogen indirectly by forming a complex with plasminogen, which subsequently behaves as a plasminogen activator to activate other plasminogen molecules by cleaving the arginyl-valine bond.

Although thrombolytic drugs, such as tissue plasminogen activator (tPA), streptokinase and urokinase, have been successfully employed clinically to reduce the extent of a thrombotic occlusion of a blood vessel, it appears that serious limitations persist with regard to their use in current thrombolytic therapy. For example, because the activation of plasminogen by tPA is fibrin dependent for full proteolytic activity to be realized (Haber et al. 1989), excessive bleeding may result as a side effect of its use. Other adverse sequelae associated with the use of these thrombolytic agents include myocardial infarction, occlusive stroke, deep venous thrombosis and peripheral arterial disease.

Additionally, the known plasminogen activators currently used suffer from several limitations that impact their overall usefulness in the elimination of a thrombus. For example, at best, the use of current thrombolytic therapy results in restored vascular blood flow within 90 min in approximately 50% of patients, while acute coronary re-occlusion occurs in roughly 10% of patients. Coronary recanalization requires on average 45 minutes or more, and intracerebral hemorrhage occurs in 0.3% to 0.7% of patients. Residual mortality is at least 50% of the mortality level in the absence of thrombolysis treatment.

A different approach to avoid the problems associated with the systemic administration of a plasminogen activator to generate sufficient plasmin at the site of the thrombus, is to directly administer the plasmin itself to the patient.

In U.S. Patent No. 5,288,489, Reich et al., disclose a fibrinolytic treatment that includes parenterally introducing plasmin into the body of a patient. The concentration and time of treatment were selected to be sufficient to allow adequate active plasmin to attain a concentration at the site of an intravascular thrombus that is sufficient to lyse the thrombus or to reduce circulating fibrinogen levels. However, the necessity of generating the plasmin from plasminogen immediately prior to its introduction into the body is also disclosed.

In contrast, U.S. Patent No. 3,950,513 to Jenson teaches that plasmin compositions may be stabilized at pH 7.0 by including a physiological non-toxic amino acid. This method dilutes stock plasmin solutions stored at low pH with the neutralizing amino acid immediately prior to administration. There are advantages, however, in maintaining low pH of the plasmin composition as long as possible to minimize autodegradation. Ideally, the plasmin will be retained at a low pH until encountering the target fibrin.

Yago et al. disclose plasmin compositions useful as a diagnostic reagent in U.S. Patent No. 5,879,923. The compositions of Yago et al. comprise plasmin and an additional component which may be 1) an oligopeptide comprising at least two amino acids, or 2) at least two amino acids, or 3) a single amino acid and a polyhydric alcohol. However, the compositions of Yago et al. are formulated at a neutral pH to maintain the enzymatic activity of plasmin.

Castellino and Sodetz describe the preparation of rabbit plasminogen and plasmin isozymes in Methods in Enzymology 45: 273-286, 1976.

Plasmin as a potential thrombolytic agent has numerous technical difficulties. These difficulties include the challenge of preparing pure plasmin that is free of all functional traces of the plasminogem activator used to convert plasmin from its inactive precursor, plasminogen. Preparations of plasmin are typically extensively contaminated by plasminogen activator, streptokinase or urokinase and the thrombolytic activity was, therefore, attributed to the contaminating plasminogen activators rather than to plasmin itself. The contaminating plasminogen activators could also trigger systemic bleeding other than at the targeted site of thrombosis. A drawback of streptokinase containing plasmin preparations is that streptokinase can cause adverse immune reactions including fever and anaphylactic shock.

One of the more important technical factors limiting clinical use of plasmin is that plasmin, as a serine protease with broad specificity, is highly prone to autodegradation and loss of activity. This circumstance provides severe challenges to the production of high-quality plasmin, to the stable formulation of this active protease for prolonged periods of storage prior to use, and to safe and effective administration of plasmin to human patients suffering from occlusive thrombi. Thus, there is need for a method of producing stable plasmin.

### Summary

The present invention provides for both a process for producing a reversibly inactive acidified plasmin by activating plasminogen and a process for producing a purified plasminogen. The produced plasmin is isolated and stored in a low pH buffering capacity agent to provide a substantially stable formulation. The purified plasminogen is typically purified from a fraction obtained in the separation of immunoglobulin from Fraction II + III by affinity chromatography with an elution at a low pH. The reversibly inactive acidified plasmin may be used in the administration of a thrombolytic therapy.

Briefly, the method for purifying plasmin comprises cleaving a plasminogen in the presence of a plasminogen activator to yield an active plasmin and removing the plasminogen activator from the active plasmin to form a plasmin solution. A low pH-buffering capacity agent can then be added to the final plasmin solution to form a reversibly inactive acidified plasmin. The final plasmin solution may be buffered to a pH of between about 2.5 to about 4.

The plasminogen activator can be removed from the active plasmin by binding the active plasmin to an active plasmin-specific absorbent material to form a bound plasmin. One such active plasmin-specific absorbent material can comprise benzamidine. Once bound, the active plasmin can be eluted with a low pH solution to form a final plasmin solution. Plasminogen activator may also be further removed by hydrophobic interaction.

A further method of purifying plasmin comprises cleaving plasminogen to yield an active plasmin and binding the active plasmin to an active plasmin-specific absorbent material to form a bound plasmin. The bound plasmin can be eluted with a substantially neutral amino acid to form a final plasmin solution which is substantially free of degraded plasmin. The substantially neutral amino acid can comprise an omega-amino acid and is typically filtered out of the final plasmin. The final plasmin may also be buffered with a low pH-buffering capacity agent.

The process for the purification of plasminogen from a plasma source includes the steps of adding the plasminogen containing solution to a plasminogen-specific absorbent material and then eluting the plasminogen from the plasminogen-specific absorbent material at a pH of between about 1 to about 4. The purified plasminogen is then collected as an eluate. Additionally, the process may include methods for the purification of micro- or mini-plasmin(ogen) or other truncated or modified forms of plasmin(ogen).

Thus, a process is now provided that successfully addresses the shortcomings of existing processes and provides distinct advantages over such processes. Additional objects, features, and advantages of the invention will become more apparent upon review of the detailed description set fourth below when taken in conjunction with the accompanying drawing figures, which are briefly described as follows.

### Brief Description of the Figures

In the Drawings:
Figure 1 graphically depicts the effect of on plasminogen recovery and lipid removal from CCI filtrate I through PEG precipitation/depth filtration;
Figure 2 graphically depicts nephelometry data for CCI extract and the subsequent filtrates I and II;
Figure 3 depicts a gel of coomassie stained reduced SDS-PAGE (10-20% Tris-Glycine) of CCI extract, filtrates and UF/DF retentate;
Figure 4 depicts a coomassie stained reduced SDS-PAGE (10-20% Tris-Glycine) of lysineSEPHAROSE 4B affinity purification of Pmg;
Figure 5 graphically depicts a lysineSEPHAROSE 4B chromatogram for the affinity purification of Pmg;
Figure 6 depicts a coomassie stained reduced SDS-PAGE (10-20% Tris-Glycine) of pH adjustment of the lysineSEPHAROSE 4B eluate (Pmg) with and without epsilon amino caproic acid present;
Figure 7 graphically represents streptokinase activation solution stability following 0.5 M NaCl, 0.25 M ε-ACA stop;
Figure 8 graphically represents benzamidine SEPHAROSE 6B chromatogram for the affinity purification of SK activated Pm;
Figure 9 depicts a coomassie stained reduced SDS-PAGE (10-20% Tris-Glycine) of benzamidine SEPHAROSE 6B purified Pm;
Figure 10 graphically depicts the hydrophobic interaction chromatography (OctylSEPHAROSE 4 FF) chromatogram for the removal of streptokinase; and

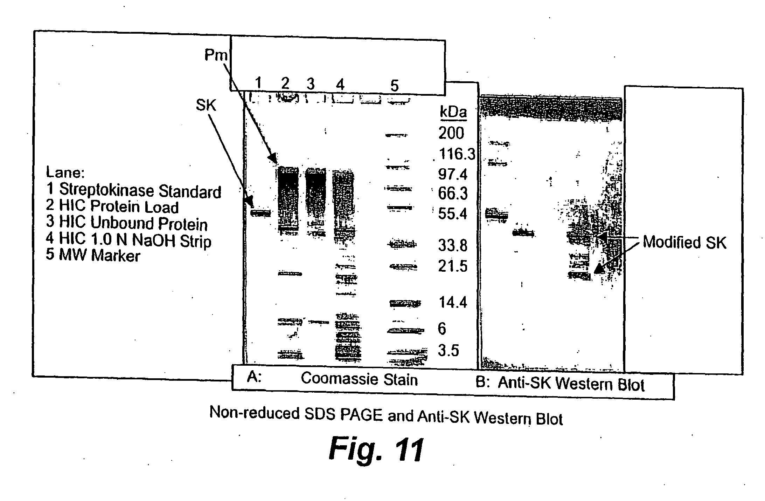

Figure 11 depicts a non-reduced SDS PAGE and anti-SK Western Blot.

### Detailed Description

The present invention comprises both a method for producing a reversibly inactive acidified plasmin in combination with a low pH-buffering capacity agent and a method for the purification of plasminogen from a plasma source. The inactive acidified plasmin solution may also include a stabiliser in addition to being inactivated in buffered solution. The process for purifying plasminogen provides for both inactivation and removal of pathogens and the elution of the plasminogen at a low pH. The inactive acidified plasmin preparation can be used in the administration of a thrombolytic therapy.

### Purification ofplasminogen

The present invention includes both a process for the purification of plasminogen and plasmin and concurrently, methods for the inactivation and removal of viral and Transmissible Spongiform Encephalopathies (TSE) contaminants during these processes. The starting material, plasminogen, can be purified from Cohn Fraction II + III paste by affinity chromatography on Lys-SEPHAROSE as described by Deutsch & Mertz (1970). SEPHAROSE is a trade name of Pharmacia, Inc. of New Jersey for a high molecular weight substance for the separation by gel filtration of macromolecules. The process may be performed on any plasma source, recombinant source, cell culture source or transgenic source. For example, plasma from a waste fraction derived from the purification of immunoglobulin from a chromatographic process can be used as described in commonly owned U.S. Patent Application Serial No. 09/448,771, filed November 24, 1999, which is incorporated by reference herein.

Plasminogen was extracted from this waste fraction (referred to herein as the "caprylate cake 1" (CCI)) over a wide range of pH. Conditions of extraction can be varied from a pH of about 3.5 to about 10.5 using a variety of buffers capable of providing a pH in this range, including citrate, acetate, tris, imidazole, histadine, HEPES and/or phosphate buffers. The extraction can occur at temperatures from about 4°C to 37°C and can be run for 1 to 24 hours without deleterious effect. In addition, the ionic strength can be varied by the addition of about 0.2 Molar sodium chloride without deleterious effect on the extraction of plasminogen.

Following the extraction of plasminogen, lipid and protein impurities and TSE were reduced by precipitation with the addition polyethylene glycol (PEG), in a range of about 1 to about 10 % weight/volume or the addition of about 80 to about 120 g/L ammonium sulfate. The PEG or ammonium sulfate precipitate was removed by depth filtration and the resulting solution placed on a lysine affinity resin column.

If desired, the solubility of plasminogen may be enhanced by the addition of omega-amino acids (lysine, arginine, tranexamic acid, or epsilon amino caproic acid, or combinations or analogues thereof) after extraction of the caprylate cake I. Solubility enhancement may be accomplished with from about 0.02 M to about 1 M omega-amino acid, preferably about 0.1 M lysine appears to be sufficient. If added, the lysine is preferably removed after the PEG or ammonium sulfate precipitation and depth filtration by diafiltration and the resulting solution placed on a lysine affinity resin column. The phrase "lysine affinity resin" is used generally for affinity resins containing lysine or its derivatives or epsilon caproic acids as the ligand. The column can be eluted with a low pH solution of approximately 1 to 4.

The protein obtained after elution from the affinity column is generally at least 80% plasminogen. The purified plasminogen is then stored at low pH in the presence of simple buffers such as glycine and lysine or omega-amino acids. Storage at low pH also provides an opportunity for viral inactivation and removal and TSE removal as determined by spiking methods. Our studies suggest that plasmin meets the most stringent requirements for 6 log clearance of non-enveloped viruses including one 4 log removal step, and 10 log clearance for enveloped viruses including two independent 4 log elimination steps. In addition to sufficient virus clearance, plasmin has greater than 6 logs of TSE infectivity removal for added safety.

The plasminogen in solution was then activated to plasmin by the addition of a plasminogen activator, which may be accomplished in a number of ways including but not limited to streptokinase, urokinase, or the use of urokinase immobilized on resin and use of streptokinase immobilized on resin. The preferred plasminogen activator is soluble streptokinase. The addition of stabilizers such as glycerol, and omega-amino acids such as lysine, poly lysine, arginine, epsilon amino caproic acid and tranexamic acid were shown to enhance the yield of plasmin.

### Purifying Plasmin

Plasmin was purified from unactivated plasminogen by affinity chromatography on resin with benzamidine as the ligand and elution with a neutral omega-amino acid solution or low pH solution. This step can remove essentially all degraded plasmin as well as the majority of the streptokinase.

As a polishing step for the removal of remaining streptokinase, hydrophobic interaction chromatography at low pH is performed. Following the HIC step, the plasmin is formulated as a sterile protein solution by ultrafiltration and diafiltration and 0.22 µm filtration.

The present method additionally includes the steps of activating plasminogen to plasmin using a plasminogen activator and then capturing the formed active plasmin on an active plasmin specific absorbent material. The bound plasmin is then eluted with a low pH buffer. The eluted plasmin is buffered with a low pH-buffering capacity agent such as an acid. Typically, the eluted plasmin is buffered to a pH of between about 2.5 to about 4.

The low buffering capacity of the acidic buffer aids in enabling the reversibly inactivated acidified plasmin to be brought up to physiological pH quickly and then activated when administered as a thrombolytic agent. Typically, the buffer is added in a concentration at which the pH of the acidified plasmin is raised to neutral pH by adding no more than about 5 times the volume of serum to the acidified plasmin.

### Cleaving the plasminogen to yield an active plasmin

Plasminogen can be cleaved to plasmin by using a catalytic concentration of an immobilized or soluble plasminogen activator. Plasmin, the principle fibrinolytic enzyme in mammals, is a serine protease with trypsin-like specificity that is derived from the inactive zymogen precursor plasminogen circulating in plasma. Plasminogen itself is a 790 amino acid polypeptide having an N-terminus glutamate residue. Plasminogen activators such as soluble streptokinase, tissue plasminogen activator (tPA) or urokinase will cleave the single-chain plasminogen molecule to produce active plasmin at the Arg560-Va1561 peptide bond. The resulting two polypeptide chains of plasmin are held together by two interchain disulfide bridges. The light chain of 25kDa carries the catalytic center and is homologous to trypsin and other serine proteases. The heavy chain (60kDa) consists of five triple-loop kringle structures with highly similar amino acid sequences. Some of these kringles contain so-called lysine-binding sites that are responsible for plasminogen and plasmin interaction with fibrin, alpha2-antiplasmin or other proteins.

The activation of plasminogen can occur at about 4°C to about 37°C and typically takes between about 2 to 24 hours. The plasminogen can be cleaved in the presence of stabilisers such as omega-amino acids and glycerol. The omega-amino acids can include lysine, epsilon amino caproic acid, tranexamic acid, poly lysine, arginine and combinations or analogues thereof. Upon the completion of the activation, the plasmin solution can be filtered and further stabilised for several days at neutral pH by the addition of omega-amino acids and sodium chloride and applied to benzamidine-SEPHAROSE.

### Removing Plasminogen Activator and Impurities

The active plasmin formed from the cleaving of the plasminogen can then be bound to an active plasmin specific absorbent to substantially remove the plasminogen activator. Since the protein of interest is an active serine protease with trypsin-like specificity, benzamidine may be used as an active plasmin specific absorbent that allows for the capture of the active plasmin. Other active plasmin specific absorbents having similar properties as benzamidine may also be used. The benzamidine can be immobilized in a solid support medium. The solid support medium can be a resin or SEPHAROSE. Additionally, hydrophobic interaction may be used to further remove the plasminogen activator.

More specifically, the cleaved plasminogen is typically contained in a solution of amino acids, sodium chloride and glycerol, which allows for stability of the solution for several days at neutral pH before it is applied to a benzamidine-SEPHAROSE column equilibrated with about 0.05 M Tris, pH 8.5, 0.5 M NaCl. The column is typically run at 4°C. The front portion of the non-bound peak contains high-molecular weight impurities, with the rest of the non-bound peak being represented by residual non-activated plasminogen and by inactive autodegradation products of plasmin.

The bound plasmin can then be eluted with an acid buffer or with a substantially neutral omega-amino acid. The plasmin bound to benzamidine-SEPHAROSE can be eluted with an acidic buffer such as glycine buffer. When a substantially neutral pH omega-amino acid is used to elute the bound plasmin the final eluted plasmin solution can be substantially free of degraded plasmin. Typically, the substantially neutral pH amino acid has a pH of value of between about 6.5 to about 8.5. Examples of neutral omega-amino acids include lysine, epsilon amino caproic acid, tranexamic acid, poly lysine, arginine, and analogues and combinations thereof.

### Buffering the plasmin solution with a low pH-buffering capacity agent

The eluted plasmin can be buffered with a low pH-buffering capacity agent. The low pH-buffering capacity agent typically comprises a buffer of either an amino acid, a derivative of at least one amino acid, an oligopeptide which includes at least one amino acid, or a combination of the above. Additionally the low pH-buffering capacity agent can comprise a buffer selected from acetic acid, citric acid, hydrochloric acid, carboxcylic acid, lactic acid, malic acid, tartaric acid, benzoic acid, serine, threonine, methionine, glutamine, alanine, glycine, isoleucine, valine, alanine, aspartic acid, derivatives or combinations thereof The buffer can be present in the reversibly inactive acidified plasmin at a concentration at which the pH of the acidified plasmin is raised to neutral pH by adding no more than about 4 to 5 times the volume of serum to the composition.

The concentration of plasmin in the buffered solution can range from about 0.01 mg/ml to about 50 mg/ml of the total solution. The concentration of the buffer can range from about 1nM to about 50mM. Of course these ranges may be broadened or narrowed depending upon the buffer chosen, or upon the addition of other ingredients such as additives or stabilizing agents. The amount of buffer added is typically that which will bring the reversibly inactive acidified plasmin solution to have a pH between about 2.5 to about 4.

### Further Stabilizing the Inactive Acidified Plasmin Solution

The reversibly inactive acidified plasmin solution may be further stabilized by the addition of a stabilizing agent such as a polyhydric alcohol, pharmaceutically acceptable carbohydrates, salts, glucosamine, thiamine, niacinamide, or combinations thereof. The stabilizing salts can be selected from the group consisting of sodium chloride, potassium chloride, magnesium chloride, calcium chloride and combinations thereof. Sugars or sugar alcohols may also be added, such as glucose, maltose, mannitol, sorbitol, sucrose, lactose, trehalose, and combinations thereof.

Concentrations of carbohydrate added to stabilize the reversibly inactive acidified plasmin solution include a range from about 0.2% w/v to about 20% w/v. Ranges for a salt, glucosamine, thiamine, niacinamide and their combinations can range from about 0.01 M to about 1 M.

The plasmin being formulated in a buffered acidified water has been found to be extremely stable. It can be kept in this form for months without any loss of activity or the appearance of degradation products of a proteolytic or acidic nature. At 4°C, plasmin is stable for at least nine months. Even at room temperature, plasmin is stable for at least two months. Long-term stability at room temperature is important because it would make this formulation compatible with long regimens of thrombolytic administration. For example, 36 hours administration of thrombolytics such as tissue plasminogen activator or urokinase is common in treatment of peripheral arterial occlusions.

The ability of a buffered acidified plasmin to become fully active upon transfer to physiological pH is evidenced by its activity in the caseinolytic assay and also in the I¹²⁵ -fibrin-labelled clot lysis assays. Both of these assays are performed at pH 7.4, and there was complete recovery of plasmin activity during the change of pH and passing through the iso-pI point (pH 5 - 5.5). This is because plasmin is formulated in a non-buffered solvent and when added to a buffered solution (either PBS of plasma) it adopts the neutral pH instantly and the precipitation that usually accompanies the slow passage through the iso-pI point, does not occur.

A feature of the active plasmin as used in the present invention is the maintenance of the plasmin in an acidic buffer and its formulation in acidified water, providing a pure and stable active plasmin. Its efficacy was demonstrated in *in vitro* assays and in an *in vivo* rabbit jugular vein thrombolysis model unified, substantially purified or partially purified enzyme such as, but not limited to, plasmin or any composition containing plasmin that is within the scope of the present invention.

The following examples are given only to illustrate the present process and are not given to limit the invention. One skilled in the art will appreciate that the examples given only illustrate that which is claimed and that the present process is only limited in scope by the appended claims.

### Examples

### Example 1

### Caprylate Cake I (CCI) Extraction and Lipid Reduction by PEG Precipitation and Filtration

Caprylate cake I (CCI) is a fraction resulting from a pH 5 caprylate precipitation of resuspended Fraction II+III in the IGIV-C process. Plasminogen (Pmg) is extracted from the CCI by solubilizing at a cake:buffer ratio of about 1:10 for 2 to 3 hours at 4°C with mixing. While several extraction solutions were investigated, the current method was performed with 100 mM Tris pH 10.5 to maintain the pH at or above neutral; a condition favorable to Pmg solubilization from the CCI. Table 1 depicts the extraction solutions investigated along with their final extract pH and Pmg potency.

**Table 1**

| CCI Extraction Solutions and Their Resulting Final Extract pHs and Pmg Activities. | | |
|---|---|---|
| Extraction Solution | Final Extract pH | Pmg (IU/mL) |
| 0.1 M Tris pH 10.5 | 9.2 - 9.5 | 1.77 |
| 0.2 M Tris pH 7.5 | 7.5 | 2.06 |
| 0.05 M Citrate, 0.2 M ε-ACA, 0.4 M NaCl pH 6.5 | 6.0 | 1.49 |
| 0.15 M Citrate pH 8.3 | 6.7 | 1.21 |
| 0.4% Acetic Acid pH 3.5 | 3.5 | 0.05 |

Following 2 to 3 hours of extraction, the temperature of the extract is adjusted to 20°C and the pH to 7.5. Table 2 shows the Pmg yield, based on nephelometry, from Clarified Plasma Pool through Fraction II+III and CCI Extract.

**Table 2**

| Step and Process Yields for Pmg from Clarified Plasma Pool to CCI Extract. | | | |
|---|---|---|---|
| Cohn Fraction | mg Pmg/g (SD), n | % Pmg Step Yield | % Pmg Process Yield |
| Clarified Plasma Pool | 0.124 (0.013), 33 | | |
| Fraction II+III | 0.143 (0.024), 30 | 65.6 | |
| CCI Extract (post L-lysine) | 0.145 (0.01), 7 | 101 | 66.3 |

Only about 66% of the Pmg in plasma tracks to Fraction II+III while virtually all of the Pmg found in the resuspended Fraction II+III precipitates to and is extracted from CCI. Extraction of CCI in Tris pH 10.5, final CCI Extract pH of 9.2 - 9.5, solubilizes all of the Pmg found in the CCI.

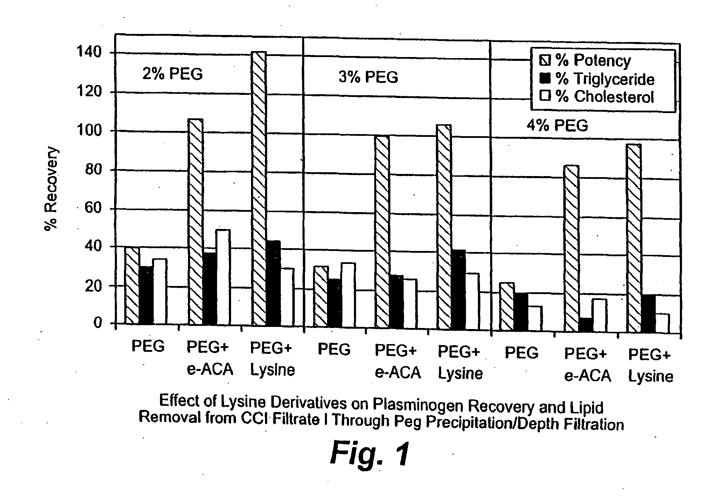

The addition of lysine derivatives (100 mM L-lysine, 50 mM epsilon amino caproic acid (EACA)) increases the solubility of Pmg in the CCI Extract resulting in increased recoveries during subsequent PEG precipitation and filtration steps as illustrated in Figure 1.

Reduction of lipid is achieved through precipitation by the addition of PEG 3350 to 3% - 4% w/w. As mentioned previously, the addition of L-lysine to 100 mM prior to PEG addition is necessary to maintain high Pmg recovery in the PEG filtrate, or about 90%. Without the addition of lysine, only about 25% of the Pmg is recovered in the PEG filtrate (Figure 1). The PEG precipitation proceeds for 1 to 2 hours at 20°C with mixing. Filter aid is added to 4% w/w and mixed prior to depth filtration through a CUNO 30SP followed by further clarification with 0.5 micron and 0.22 micron filters.

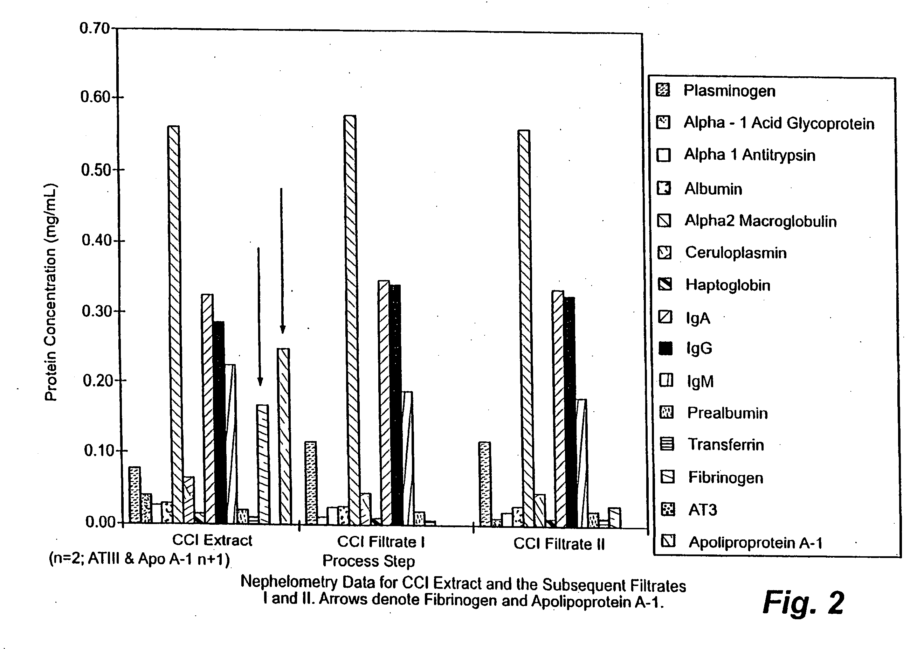

Figure 1 shows the lipid content, determined by cholesterol and triglycerides concentration, is reduced by 60 - 70% following PEG precipitation and filtration (CCI Filtrate I). The CCI Filtrate I is diluted 1:1 with phosphate buffered saline pH 7.5 and held at 20°C for 1 to 2 hours as precipitation often continues following filtration. The CCI Filtrate I is filtered through 0.5 µm and 0.22 µm filters to remove any additional precipitate; CCI Filtrate II. Nephelometry data for CCI Extract and CCI Filtrates I and II are illustrated in Figure 2. Note that fibrinogen and apolipoprotein A-1 concentrations are reduced following PEG precipitation.

The CCI Filtrate II is diafiltered by tangential flow filtration (TFF) against phosphate buffered saline pH 7.5 to reduce the L-lysine concentration such that it will not act as a competitive inhibitor for Pmg binding to the lysine affinity resin. Experiments were performed to illustrate the necessity of lysine removal. Loading the CCI Filtrate II directly onto a lysine affinity resin without reduction in soluble lysine concentration, results in the capture and release of about 4% of the Pmg activity. Diluting the CCI Filtrate II 1:1 with TBS (10 mM Tris, 150 mM NaCl pH 7.5) still resulted in capture and release of only about 5% of the Pmg activity. Following 5 volumes of diafiltration to reduce the lysine concentration, about 22% of the Pmg activity was captured and released from the lysine affinity resin (in retrospect, the column was overloaded by about 50%).

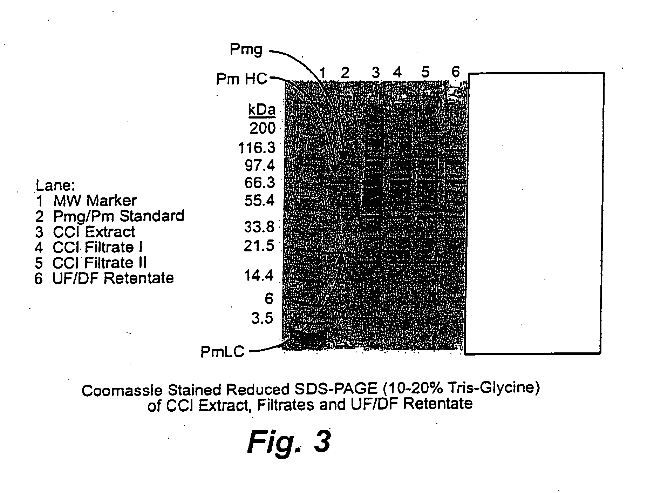

Constant volume diafiltration was performed by tangential flow filtration (TFF) against 5 volumes phosphate buffered saline pH 7.5 using a 30 kDa molecular weight cutoff membrane. Following diafiltration, the protein solution was concentrated by ultrafiltration to 4 to 5 A₂₈₀/mL. Pmg recoveries in the UF/DF retentate, by nephelometry, averaged 84% (±1, n=3). Figure 3 shows reduced SDS PAGE for each of the process intermediates discussed thus far. The data in Figures 2 and 3 illustrate the complexity and heterogeneity of the CCI Extract and subsequent Filtrates.

### Example 2

### Purification of Pmg by Lysine Affinity Chromatography:

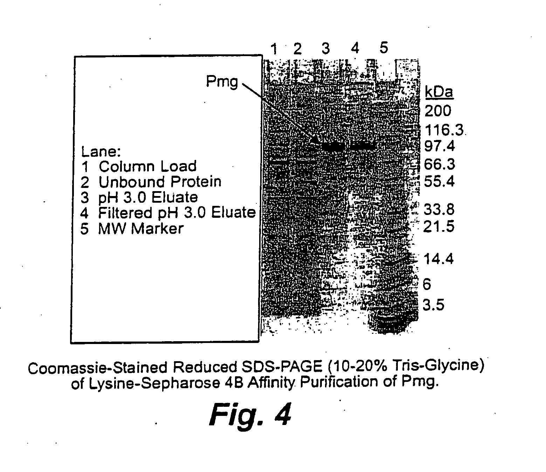

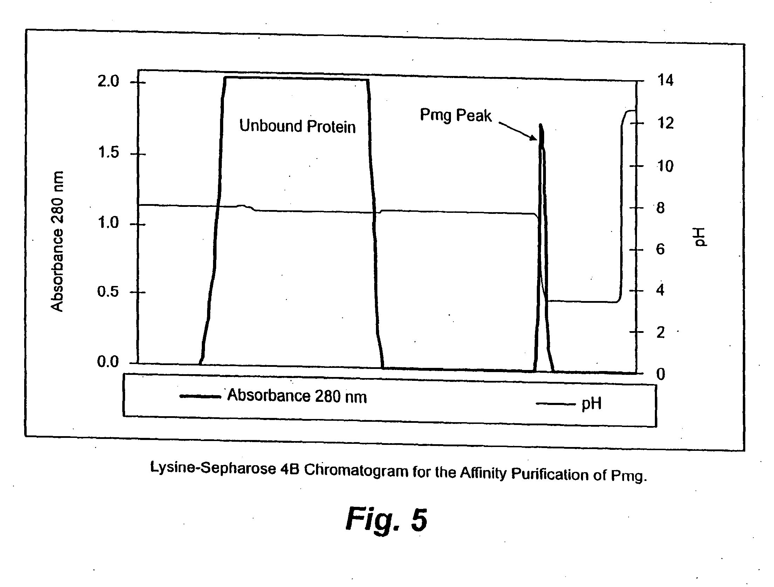

The purpose of lysine affinity chromatography is to purify Pmg, which represents from about 3 to 5% of the total protein in the diafiltered CCI Filtrate II. The DF CCI Filtrate II was applied to a Lysine-SEPHAROSE 4B (Amersham Pharmacia #17-0690-01) column equilibrated with 0.01 M NaH₂PO₄, 0.15 M NaCl pH 7.5, at 3.5-4.0 A₂₈₀/mL resin. Unbound proteins were washed through the column with the equilibration buffer and the resin was then washed with 0.01 M NaH₂PO₄, 0.5 M NaCl pH 7.5 to remove non-specifically bound protein; no protein was removed. Bound protein, Pmg, was eluted with 0.1 M Glycine, 0.03 M Lysine pH 3.0 and collected with mixing to maintain low pH. Figures 4 and 5 show SDS PAGE analysis and the chromatogram of the lysine affinity purification of Pmg, respectively The resin was cleaned sequentially with 0.1 N NaOH and 2.0 M NaCl, 0.1% Triton X-100 and stored in 20% ethanol. Table 3 shows Pmg step yield by nephelometry and purity by reduced SDS PAGE.

**Table 3**

| Lysine Affinity Chromatography Pmg Step Yield and Purity | | |
|---|---|---|
| Process Intermediate | Step Yield % | Pmg Purity % |
| Lysine-SEPHAROSE 4B Eluate | 75.7 | 85.9 |

### Example 3

### Viral Inactivation and Removal and TSE Removal

### Nanofiltration

The optimal placement of a nanofiltration step during the Plasmin process, along with determining the optimal conditions for pathogens removal from Pmg lysine affinity eluate (Pmg) for a particular nanofiltration scheme was tested. Pmg was spiked with PPV or BVDV and filtered through a PALL DV20 filter membrane. All runs were performed with 50 ml starting material (0.3 mg/ml Pmg), 30 psi constant pressure, pH 3.4 and room temperature. The challenge solution was prefiltered through 0.22 µm prior to nanofiltration. The determining factors for the optimal conditions for removal of different pathogens by nanofiltration deal mainly with the attainment of a minimum of 4 log infectivity removal of known pathogens, percent product recovery, percent potency remaining, product concentration and product pH. We detected, that PPV and BVDV clearance was > 4 log₁₀ TCID₅₀. The nanofiltration step has also the capability of removing greater than 4 log of TSE. All product recoveries obtained in the study were ≥95% with no substantial change in Pmg activity.

### Caprylate Viral Inactivation.

Since caprylate inactivation is very much pH dependent and more efficacious under acidic pH conditions, it was logical to study virus inactivation by caprylate at the low pH lysine affinity chromatography elution step. We used BVDV as a model enveloped virus to study caprylate virucidal activity in lysine affinity eluate. Complete BVDV inactivation, resulting in ≥4.4 log₁₀ reduction, was detected at the lysine affinity column eluate with 3mM caprylate at pH 3.4 during 30 min of incubation at room temperature in the presence of 1.5 mg/ml Pmg. In the absence of product, complete BVDV inactivation (≥4.7 log₁₀ reduction) was also achieved with 3mM caprylate after 30 minutes at pH 3.4. No visible precipitation was observed during the caprylate treatment suggesting that the product and virus spike remain soluble and are not being precipitated by the caprylate. The impact of the added caprylate on product recovery or potency following lysine affinity column chromatography was minimal.

### PEG Precipitation

We have investigated the effect of PEG on TSE removal. The clarification and removal of lipids achieved by depth filtration and 3% PEG precipitation of the Caprylate Cake I Extract resulted in greater than 2 log₁₀ of TSE removal.

**Table 4**

| Total Virus/TSE clearance across Plasmin process | | | |
|---|---|---|---|
| Step | BVDV | PPV | TSE |
| Nanofiltration | >4 log | 4 log | 4 log |
| 3mM Caprylate | >4 log | <1 log | <1 log |
| Lysine Affinity | 3.3 log | 2.5 log | pending |
| PEG precipitation | <1 | <1 | 2-3 logs |
| Total clearance | >12 | >6 | >6 |

### Example 4

### Streptokinase (SK) Activation of Pmg to Pm (Pm):

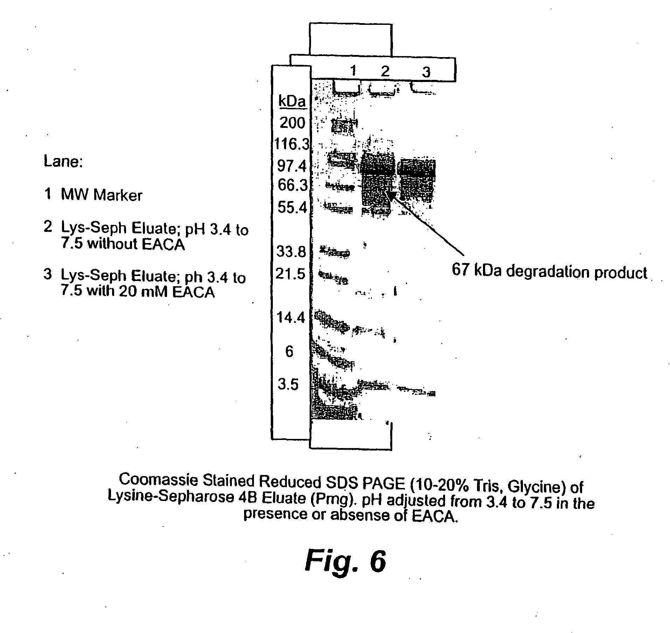

The addition of SK to the purified Pmg solution effects the conversion of Pmg to Pm. The lysine affinity column eluate pH 3.4 is concentrated by TFF to 2 mg/mL through a 30 kD molecular weight cutoff membrane. The Pmg solution temperature is ramped down to 4°C and a Pmg stabilizer, EACA, is added to a final concentration of 20 mM to protect Pmg against damage during pH adjustment from 3.4 to 7.5. Without the addition of EACA, a 67 kDa species appears following the pH swing. The presence of EACA during pH adjustment results in decreased Pmg degradation as compared to pH adjustment without EACA (Figure 6). Once the pH is adjusted to 7.5, the Pmg solution is diluted 1:1 with 20% glycerol, 4°C, to achieve a final condition of 1 mg Pmg/mL 0.05 M glycine, 0.015 M L-lysine, 0.01 M EACA, 10 % glycerol pH 7.5. These conditions have been optimized for minimizing Pm autodegradation. SK is added to this solution at a 100:1 Pmg:SK molar ratio. The SK reaction mixture is mixed at 4°C for 16 hours to allow activation of Pmg to Pm. The average relative percent purity, as determined by reduced SDS PAGE, of each of 4 groups of protein species (Pmg, Pm HC, Pm LC and impurities/clipped Pm) from 14 SK activation reactions are listed in Table 5.

**Table 5**

| Relative Average % of Pmg, Pm (HC, LC) and Impurities/Clipped Pm by Reduced SDS PAGE Following SK Activation; n=14. | | |
|---|---|---|
| Protein | Average % Purity | SD |
| Pmg | 20.3 | 5.3 |
| Pm | 68.5 | 4.4 |
| Pm Heavy Chain | 49.0 | 2.9 |
| Pm Light Chain | 19.4 | 1.5 |
| Impurities/Clipped Pm | 11.3 | 1.8 |

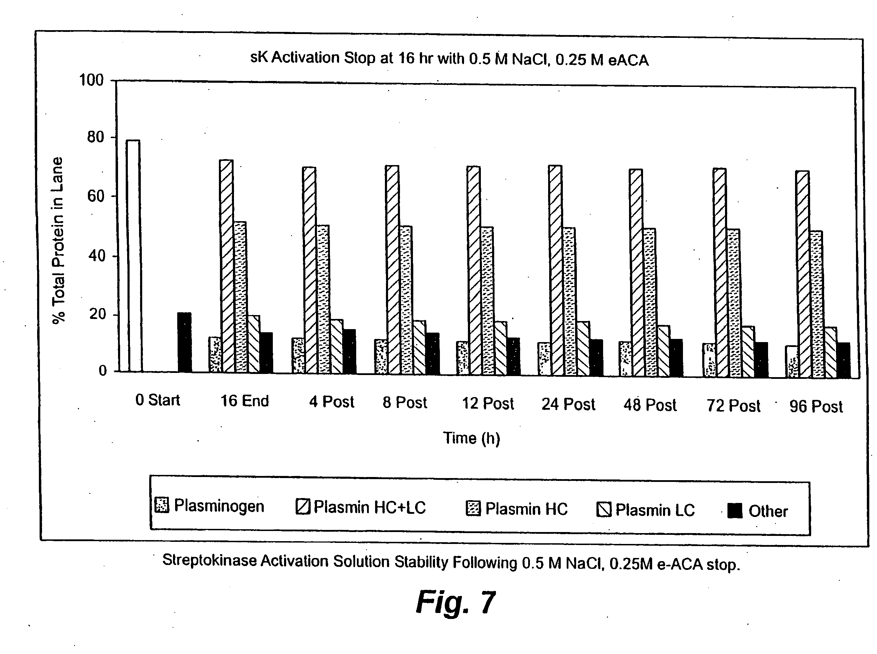

The data shows that the SK activation is reproducible and results in only about 11% clipped Pm/impurities while activation of Pmg to Pm is about 80%. To stop the activation and Pm autodegradation reactions, NaCl and EACA are added to final concentrations of 0.5 M and 0.25 M, respectively. This solution is stable with respect to Pm integrity, for at least 4 days at 4°C. Figure 7 illustrates that there is no change in the Pm purity or Pm autodegradation (Other) over this time period.

### Example 5

### Purification of Pm by Benzamidine Affinity Chromatography:

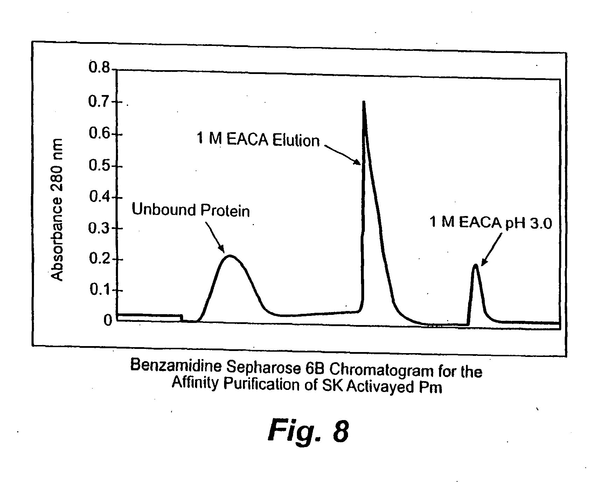

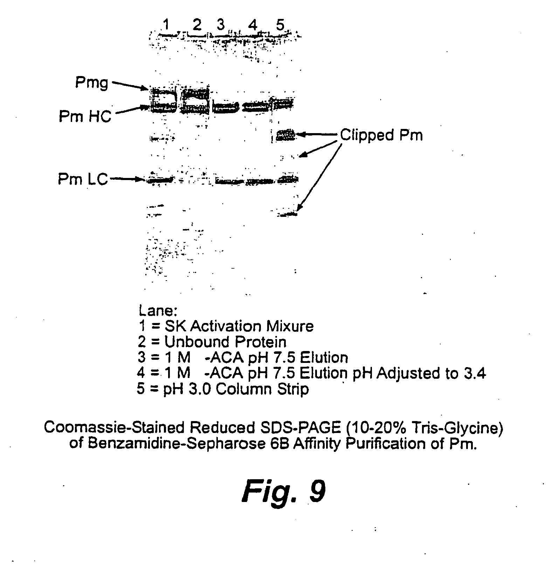

The purpose of benzamidine affinity purification is the separation of unactivated Pmg and impurities, including Pm degradation products, from active Pm. The stable SK activation solution, pH adjusted to 8.5 in 0.05 M glycine, 0.015 M L-lysine, 0.25 M EACA, 0.5 M NaCl, 10 % glycerol, is applied to a Benzamidine-SEPHAROSE 6B (Amersham Pharmacia #17-0568-01) column equilibrated with 50 mM Tris, 500 mM NaCl, pH 8.5. The Pm, both clipped and intact, is captured by the affinity resin while the aforementioned impurities flow through the column. The column is washed with the equilibration buffer until the absorbance at 280 nm reaches baseline. The bound Pm is then eluted in either one of two ways: 1) removing the resin and eluting in batch format with 0.1 M Glycine, 0.03 M Lysine pH 3.4; 2) eluting in a column format with 1 M EACA pH 7.5. Elution with EACA pH 7.5 removes only the intact Pm while damaged Pm remains bound to the resin. A step to strip all remaining protein. Figure 8 shows a typical column format, EACA elution profile. The batch elution profile consists only of the unbound protein peak as the resin is then removed from the column for Pm elution. The Pm captured and eluted from the affinity resin is 87 - 91 % intact (non-autodegraded) as illustrated in Figure 9 and ≥ 99% total Pm. The elution of Pm from the benzamidine resin with EACA was unexpected as lysine derivatives such as EACA interact with the heavy chain of Pm while benzamidine interacts with the light chain.

### Example 6

### Removal of the Pmg Activator SK

The purpose of these steps is to remove the Pmg activator SK such that the only remaining fibrin clot dissolution activity is that of Pm. The benzamidine affinity step removes >99% of the SK from the Pm as is illustrated in Table 6.

**Table 6**

| SK removal, as determined by ELISA, by benzamidine affinity chromatography and hydrophobic interaction chromatography. | |
|---|---|
| Plasmin Process Step | Streptokinase (ng/mL) |
| SK activation | 1930.1 |
| Benzamidine-SEPHAROSE unbound | 1549.5 |
| Benzamidine-SEPHAROSE eluted Pm | 1.9 |
| HIC Unbound Pm | 0.7 |
| HIC NaOH strip (SK) | 1.3 |
| Final Formulation Pm | <0.5 |

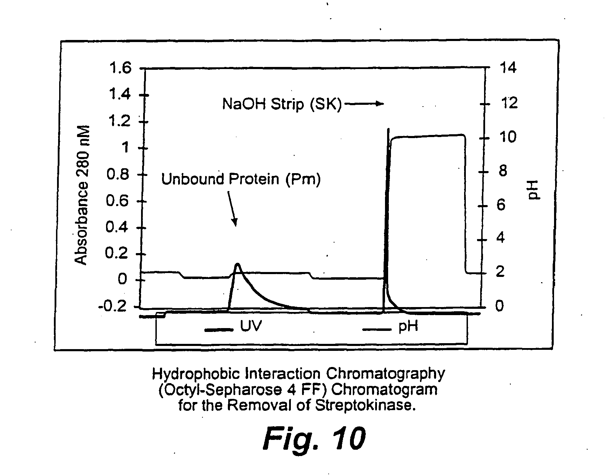

The hydrophobic interaction step using Octyl SEPHAROSE 4 FF (Amersham Pharmacia #17-0946-02) acts as a polishing step to remove essentially any remaining SK. The final sterile Pm product has no detectable SK by ELISA. The 1 M EACA eluate pH 7.5, from the benzamidine affinity column, is adjusted to pH 3.4 and (NH₄)₂SO₄ is added to a final concentration of 0.1 M. This acts as the protein load for the Octyl-SEPHAROSE 4 FF column. The column is equilibrated with 0.1 M (NH₄)₂SO₄, 0.1 M Glycine, 30 mM Lysine pH 3.4. Pm flows through the column while SK binds to the column and is separated from Pm. The captured SK is removed from the resin along with 0.1 to 1.0 N NaOH. Figure 9 is an Octyl-SEPHAROSE 4 FF chromatogram from a proof of principle experiment. Pmg and SK were mixed at a 2:1 Pmg:SK molar ratio and subjected to Octyl-SEPHAROSE 4 FF chromatography. The high levels of SK were used so it could be tracked throughout the chromatographic cycle using an anti-SK western blot. Figure 10 illustrates the removal of SK from the Pm by SDS PAGE and anti-SK western blot. The SK standard (panels A and B; lane 1) migrates true to its molecular weight of 47 kDa. Once mixed with Pmg, the SK is modified and migrates faster and as several species. There is no detectable SK in the unbound protein fraction, which contains the bulk of the Pm, by anti-SK western blot (panel B; lane 3).

Results for final sterile preparations of Pm purified by benzamidine affinity and HIC chromatographies, as described above, are listed in Table 7.

**Table 7**

| Relative Average % Purity of Pm (HC, LC) by Reduced SDS PAGE Following HIC; n=2. | |
|---|---|
| Protein | Average % Purity |
| Pmg | 0.0 |
| Pm | 95.5 |
| Pm Heavy Chain | 66.5 |
| Pm Light Chain | 29.0 |
| Impurities/Clipped Pm | 4.5 |

While specific embodiments have been set forth as illustrated and described above, it is recognized that variations may be made with respect to disclosed embodiments.

## Claims

1. A method for purifying plasmin comprising:
cleaving a plasminogen in the presence of a plasminogen activator to yield an active plasmin;
substantially removing the plasminogen activator from the active plasmin to form a plasmin solution; and
buffering the plasmin solution with a low pH-buffering capacity agent to form a reversibly inactive acidified plasmin,
wherein the low pH-buffering capacity agent is present at a concentration at which the pH of the acidified plasmin is raised to neutral pH by adding no more than 5 times the volume of serum to the acidified plasmin.

2. The method of claim 1, wherein the step of substantially removing the plasminogen activator includes the steps of:
binding the active plasmin to an active plasmin-specific absorbent material to form a bound plasmin; and
eluting the bound plasmin with a low pH solution to form the plasmin solution.

3. The method of claim 2, wherein the active plasmin-specific absorbent material comprises benzamidine.

4. The method of claim 2, wherein the plasminogen activator is further removed by hydrophobic interaction.

5. The method of claim 1, further including cleaving the plasminogen in the presence of stabilizers comprising omega-amino acids and glycerol.

6. The method of claim 1, wherein the plasminogen is cleaved using a catalytic concentration of a plasminogen activator that is selected from the group consisting of immobilized, soluble and combinations thereof of plasminogen activators.

7. The method of claim 6; wherein the plasminogen activator is selected from the group consisting of streptokinase, urokinase, tPA and combinations thereof.

8. The method of claim 7, wherein the plasminogen activator is soluble streptokinase.

9. The method of claim 6, wherein the plasminogen activator is immobilized on a solid support medium comprising SEPHAROSE.

10. The method of claim 1, wherein the low pH-buffering capacity agent comprises a buffer comprising an amino acid, a derivative of at least one amino acid, an oligopeptide which includes at least one amino acid, or a combination thereof.

11. The method of claim 1, wherein the low pH-buffering capacity agent comprises a buffer selected from acetic acid, citric acid, hydrochloric acid, carboxylic acid, lactic acid, malic acid, tartaric acid, benzoic acid, serine, threonine, methionine, glutamine, alanine, glycine, isoleucine, valine, alanine, aspartic acid, derivatives thereof, or combinations thereof.

12. The method of claim 1, wherein the reversibly inactive acidified plasmin solution has a pH between 2.5 to about 4.

13. The method of claim 1, further including stabilizing the reversibly inactive acidified plasmin by adding a stabilizing agent selected from a polyhydric alcohol, pharmaceutically acceptable carbohydrates, salts, glucosamine, thiamine, niacinamide, or combinations thereof.

14. The method of claim 13, wherein the salts are selected from the group consisting of sodium chloride, potassium chloride, magnesium chloride, calcium chloride and combinations thereof:

15. The method of claim 1, further including stabilizing the reversibly inactive acidified plasmin by adding a sugar or sugar alcohol selected from glucose, maltose, mannitol, sorbitol, sucrose, lactose, trehalose, or combinations thereof.

16. The method of claim 2, wherein eluting the bound plasmin is achieved by eluting the bound plasmin with a amino acid at a pH of 6.5 to 8.5 to form a final plasmin solution which is substantially free of degraded plasmin.

17. The method of claim 16, wherein the plasminogen is cleaved using a catalytic concentration of a plasminogen activator.

18. The method of claim 16, wherein the activated plasmin solution is stabilized by the addition of omega-amino acids and sodium chloride.

19. The method of claim 16, wherein said amino acid comprises an omega-amino acid.

20. The method of claim 19, wherein the omega-amino acid selected from the group consisting of lysine, epsilon amino caproic acid, tranexamic acid, poly lysine, arginine, analogues thereof and combinations thereof.

21. The method of claim 16, further including filtering out said amino acid from the final plasmin solution.

22. The method of claim 16, further including adding a low pH-buffering capacity agent to the final plasmin solution to form a reversibly inactive acidified plasmin.

23. The method of claim 22, further including adjusting the pH of the reversibly inactive acidified plasmin to a pH between 2.5 to about 4.

24. The method of claim 16, further including adding a stabilizer to the final plasmin solution.

25. The method of claim 24, wherein the stabilizer is selected from the group consisting of amino acids, salts or combinations thereof.

## Patentansprüche

1. Verfahren zur Reinigung von Plasmin, umfassend:
Spalten eines Plasminogens in der Gegenwart eines Plasminogenaktivators, um aktives Plasmin zu erhalten;
im wesentlichen Entfernen des Plasminogenaktivators aus dem aktiven Plasmin, um eine Plasminlösung zu bilden; und
Puffern der Plasminlösung mit einem Mittel, das eine Pufferkapazität im niedrigen pH-Bereich aufweist, um ein reversibel inaktives angesäuertes Plasmin zu bilden,
worin das Mittel mit Pufferkapazität im niedrigen pH-Bereich in einer Konzentration vorhanden ist, bei welcher der pH des angesäuerten Plasmins durch Hinzufügen von nicht mehr als dem 5-fachen Volumen von Serum zum angesäuerten Plasmin auf einen neutralen pH angehoben wird.

2. Verfahren gemäß Anspruch 1, worin der Schritt des im wesentlichen Entfernens des Plasminogenaktivators die Schritte umfaßt:
Binden des aktiven Plasmins an ein für aktives Plasmin spezifisches adsorbierendes Material, um gebundenes Plasmin zu bilden; und
Eluieren des gebundenen Plasmins mit einer Lösung, die einen niedrigen pH aufweist, um die Plasminlösung zu bilden.

3. Verfahren gemäß Anspruch 2, worin das adsorbierende Material, das spezifisch ist für aktives Plasmin, Benzamidin umfaßt.

4. Verfahren gemäß Anspruch 2, worin der Plasminogenaktivator des weiteren entfernt wird durch hydrophobe Interaktion.

5. Verfahren gemäß Anspruch 1, des weiteren umfassend Spalten des Plasminogens in der Gegenwart eines Stabilisators umfassend Omega-Aminosäuren und Glycerol.

6. Verfahren gemäß Anspruch 1, worin das Plasminogen gespalten wird unter Verwendung einer katalytischen Konzentration eines Plasminogenaktivators, der ausgewählt ist aus der Gruppe bestehend aus Plasminogenaktivatoren, die immobilisiert sind, löslich sind und Kombinationen davon.

7. Verfahren gemäß Anspruch 6, worin der Plasminogenaktivator ausgewählt ist aus der Gruppe bestehend aus Streptokinase, Urokinase, tPA und Kombinationen davon.

8. Verfahren gemäß Anspruch 7, worin der Plasminogenaktivator lösliche Streptokinase ist.

9. Verfahren gemäß Anspruch 6, worin der Plasminogenaktivator immobilisiert ist auf einem festen Trägermedium umfassend SEPHAROSE.

10. Verfahren gemäß Anspruch 1, worin das Mittel mit Pufferkapazität im niedrigen pH-Bereich einen Puffer umfaßt, umfassend eine Aminosäure, ein Derivat von zumindest einer Aminosäure, ein Oligopeptid, das zumindest eine Aminosäure umfaßt, oder Kombinationen davon.

11. Verfahren gemäß Anspruch 1, worin das Mittel mit Pufferkapazität im niedrigen pH-Bereich einen Puffer umfaßt, ausgewählt aus Essigsäure, Zitronensäure, Salzsäure, Carboxylsäure, Milchsäure, Apfelsäure, Weinsäure, Benzoesäure, Serin, Threonin, Methionin, Glutamin, Alanin, Glycin, Isoleucin, Valin, Alanin, Aspartat, Derivate davon oder Kombinationen davon.

12. Verfahren gemäß Anspruch 1, worin die Lösung von reversibel inaktivem angesäuertem Plasmin einen pH zwischen 2,5 und etwa 4 aufweist.

13. Verfahren gemäß Anspruch 1, des weiteren umfassend Stabilisieren des reversibel inaktiven angesäuerten Plasmins durch Hinzufügen eines Stabilisierungsmittels, ausgewählt aus einem polyhydrischen Alkohol, pharmazeutisch akzeptablen Kohlenwasserstoffen, Salzen, Glucosamin, Thiamin, Niacinamid oder Kombinationen davon.

14. Verfahren gemäß Anspruch 13, worin die Salze ausgewählt sind aus der Gruppe bestehend aus Natriumchlorid, Kaliumchlorid, Magnesiumchlorid, Calciumchlorid und Kombinationen davon.

15. Verfahren gemäß Anspruch 1, des weiteren umfassend Stabilisieren des reversibel inaktiven angesäuerten Plasmins durch Hinzufügen eines Zuckers oder Zuckeralkohols, ausgewählt aus Glucose, Maltose, Mannitol, Sorbitol, Sucrose, Lactose, Trehalose oder Kombinationen davon.

16. Verfahren gemäß Anspruch 2, worin das Eluieren des gebundenen Plasmins erreicht wird durch Eluieren des gebundenen Plasmins mit einer Aminosäure bei einem pH von 6,5 bis 8,5, um eine endgültige Plasminlösung zu bilden, die im wesentlichen frei ist von degradiertem Plasmin.

17. Verfahren gemäß Anspruch 16, worin das Plasminogen unter Verwendung einer katalytischen Konzentration eines Plasminogenaktivators gespalten wird.

18. Verfahren gemäß Anspruch 16, worin die Lösung von aktiviertem Plasmin durch das Hinzufügen von Omega-Aminosäuren und Natriumchlorid stabilisiert wird.

19. Verfahren gemäß Anspruch 16, worin die besagte Aminosäure eine Omega-Aminosäure umfaßt.

20. Verfahren gemäß Anspruch 19, worin die Omega-Aminosäure ausgewählt ist aus der Gruppe bestehend aus Lysin, Epsilon-amin-kaproischer Säure, Tranexaminsäure, Polylysin, Arginin, Analoge davon und Kombinationen davon.

21. Verfahren gemäß Anspruch 16, des weiteren umfassend das Herausfiltern von besagter Aminosäure aus der endgültigen Plasminlösung.

22. Verfahren gemäß Anspruch 16, des weiteren umfassend Hinzufügen eines Mittels mit Pufferkapazität im niedrigen pH-Bereich zu der endgültigen Plasminlösung, um ein reversibel inaktives angesäuertes Plasmin zu bilden.

23. Verfahren gemäß Anspruch 22, des weiteren umfassend Einstellen des pHs des reversibel inaktiven angesäuerten Plasmins auf einen pH zwischen 2,5 bis etwa 4.

24. Verfahren gemäß Anspruch 16, des weiteren umfassend Hinzufügen eines Stabilisators zu der endgültigen Plasminlösung.

25. Verfahren gemäß Anspruch 24, worin der Stabilisator ausgewählt ist aus der Gruppe bestehend aus Aminosäuren, Salzen oder Kombinationen davon.

## Revendications

1. Procédé de purification de plasmine comprenant les étapes consistant à :
cliver un plasminogène en présence d'un activateur de plasminogène pour obtenir une plasmine active ;
enlever substantiellement l'activateur de plasminogène de la plasmine active pour former une solution de plasmine ; et
tamponner la solution de plasmine avec un agent ayant une capacité tampon à faible pH pour former une plasmine acidifiée inactive de manière réversible,
dans lequel l'agent ayant une capacité tampon à faible pH est présent à une concentration à laquelle on augmente le pH jusqu'au pH neutre en n'ajoutant pas plus de 5 fois le volume de sérum à la plasmine acidifiée.

2. Procédé selon la revendication 1, dans lequel l'étape consistant à enlever substantiellement l'activateur de plasminogène comprend les étapes consistant à :
lier la plasmine active à un matériau absorbant spécifique de la plasmine activée pour former une plasmine liée ; et
éluer la plasmine liée avec une solution à faible pH pour former la solution de plasmine.

3. Procédé selon la revendication 2, dans lequel le matériau absorbant spécifique de la plasmine activée comprend la benzamidine.

4. Procédé selon la revendication 2, dans lequel l'activateur de plasminogène est en outre retiré par interaction hydrophobe.

5. Procédé selon la revendication 1, comprenant en outre le clivage du plasminogène en présence de stabilisants comprenant des oméga aminoacides et du glycérol.

6. Procédé selon la revendication 1, dans lequel le plasminogène est clivé en utilisant une concentration catalytique d'un activateur de plasminogène qui est choisi dans le groupe constitué d'activateurs de plasminogène immobilisés, solubles et de leurs combinaisons.

7. Procédé selon la revendication 6, dans lequel l'activateur de plasminogène est choisi dans le groupe constitué de la streptokinase, l'urokinase, le tPA et leurs combinaisons.

8. Procédé selon la revendication 7, dans lequel l'activateur de plasminogène est la streptokinase soluble.

9. Procédé selon la revendication 6, dans lequel l'activateur de plasminogène est immobilisé sur un milieu support solide comprenant du SEPHAROSE.

10. Procédé selon la revendication 1, dans lequel l'agent ayant une capacité tampon à faible pH comprend un tampon contenant un aminoacide, un dérivé d'au moins un aminoacide, un oligopeptide qui comprend au moins un aminoacide, ou une combinaison de ceux-ci.

11. Procédé selon la revendication 1, dans lequel l'agent ayant une capacité tampon à faible pH comprend un tampon choisi parmi l'acide acétique, l'acide citrique, l'acide chlorhydrique, l'acide carboxylique, l'acide lactique, l'acide malique, l'acide tartrique, l'acide benzoïque, la sérine, la thréonine, la méthionine, la glutamine, l'alanine, la glycine, l'isoleucine, la valine, l'alanine, l'acide aspartique, leurs dérivés, ou leurs combinaisons.

12. Procédé selon la revendication 1, dans lequel la solution de plasmine acidifiée inactivée de manière réversible a un pH entre 2,5 et environ 4.

13. Procédé selon la revendication 1, comprenant en outre la stabilisation du la plasmine acidifiée inactive de manière réversible en ajoutant un agent stabilisant choisi parmi un alcool polyhydroxylé, des glucides pharmaceutiquement acceptables, des sels, la glucosamine, la thiamine, le niacinamide, ou des combinaisons de ceux-ci.

14. Procédé selon la revendication 13, dans lequel les sels sont choisis dans le groupe constitué du chlorure de sodium, chlorure de potassium, chlorure de magnésium, chlorure de calcium et leurs combinaisons.

15. Procédé selon la revendication 1, comprenant en outre la stabilisation du la plasmine acidifiée inactive de manière réversible en ajoutant un sucre ou un sucre alcool choisi parmi le glucose, maltose, mannitol, sorbitol, saccharose, lactose, tréhalose, ou leurs combinaisons.

16. Procédé selon la revendication 2, dans lequel on effectue l'élution de la plasmine liée en éluant la plasmine liée avec un aminoacide à un pH de 6,5 à 8,5 pour former une solution de plasmine finale qui est essentiellement exempte de plasmine dégradée.

17. Procédé selon la revendication 16, dans lequel le plasminogène est clivé en utilisant une quantité catalytique d'un activateur de plasminogène.

18. Procédé selon la revendication 16, dans lequel la solution de plasmine active est stabilisée par l'addition d'oméga aminoacides et de chlorure de sodium.

19. Procédé selon la revendication 16, dans lequel ledit aminoacide comprend un oméga aminoacide.

20. Procédé selon la revendication 19, dans lequel l'oméga aminoacide est choisi dans le groupe constitué de la lysine, l'acide epsilon aminocaproïque, l'acide tranéxamique, la polylysine, l'arginine, leurs analogues et leurs combinaisons.

21. Procédé selon la revendication 16, comprenant en outre la séparation par filtration dudit aminoacide de la solution finale de plasmine.

22. Procédé selon la revendication 16, comprenant en outre l'ajout d'un agent ayant une capacité tampon à faible pH à la solution finale de plasmine pour former une plasmine acidifiée inactive de manière réversible.

23. Procédé selon la revendication 22, comprenant en outre l'ajustement du pH de la plasmine acidifiée inactive de manière réversible à un pH entre 2,5 et environ 4.

24. Procédé selon la revendication 16, comprenant en outre l'ajout d'un stabilisant à la solution finale de plasmine.

25. Procédé selon la revendication 24, dans lequel le stabilisant est choisi dans le groupe constitué des aminoacides, de leurs sels ou de leurs combinaisons.
